# EUROPEAN PATENT APPLICATION

(11) **EP 4 144 387 A1**
(43) Date of publication of application: **08.03.2023**
(21) Application number: 20933365.7
(22) Date of filing: 30.10.2020
(51) Int. Cl.: A61L 31/02, A61L 31/16, C22C 38/38, C22C 38/22

(54) **HIGH-NITROGEN NICKEL-FREE AUSTENITIC STAINLESS STEEL SEAMLESS THIN-WALLED TUBE**

(30) Priority: 30.04.2020 CN 202010364623; 30.04.2020 CN 202010367220; 30.04.2020 CN 202010367246
(71) Applicant: Zhong Ke Yi An Medical Technology (Beijing) Co., Ltd, Beijing 101318 (CN)
(72) Inventor: LI, Wen, Beijing 101318 (CN); BAI, Shugong, Beijing 101318 (CN)
(74) Representative: Kilger, Ute
(86) International application number: PCT/CN2020/125095
(87) International publication number: WO 2021/218089

(57) **Abstract**

A high-nitrogen nickel-free austenitic stainless steel seamless thin-walled tube, a high-safety nickel-free metal-based drug-eluting vascular stent manufactured therefrom, and manufacturing methods therefor. In the process of manufacturing a stent tube, the nitrogen content of a material is further increased by means of stage-by-stage nitriding, so as to obtain a high-nitrogen nickel-free austenitic stainless steel seamless thin-walled tube having the nitrogen content of 0.8-1.2% as a metal stent platform material. By using rolling line contact type electrochemical polishing, the surface of the stent forms a micron-scale protrusion-recess structure by means of crystal grains having different orientations, thus improving a binding force between a metal stent material and a drug coating. The vascular stent has the characteristics of high fatigue life, high biological safety, and a high binding force between the drug coating and a substrate.

## Description

### TECHNICAL FIELD

The present invention relates to the field of high-nitrogen steel tubes, in particular to a high-nitrogen nickel-free austenitic stainless steel seamless thin-walled tube and a preparation method thereof. The preparation method can be applied to the preparation of chromium-manganese-nitrogen based stainless steel thin-walled tubes. In addition, the present invention relates to the field of medical devices, in particular to a nickel-free metal drug-eluting vascular stent with long service life and high safety and a manufacturing method thereof.

### BACKGROUND ART

Nickel is an essential trace element for the human body, but excessive intake can cause allergies, deformities, cancer and other pathologies. In response to the harms of nickel, many countries have become more and more stringent on the content of nickel in daily use and medical metal materials. The European Parliament and Council Directive (94/27/EC) promulgated in 1994 stipulates that the nickel content in the materials implanted in the human body should not exceed 0.05%; and for the alloys (jewelry, watches, rings, bracelets, etc.) that are in contact with human skin for a long time, the amount of nickel permeating the skin should not exceed 0.5 µg/cm² per week. In view of the harms of nickel to the human body, the research and development of medical low-nickel and nickel-free austenitic stainless steel has become a major development trend of medical stainless steel in the world.

Chromium-manganese-nitrogen based high-nitrogen nickel-free austenitic stainless steel is a stable austenitic stainless steel obtained though increasing the solid solubility of nitrogen by increasing the manganese content in the material. It has the characteristics such as high strength and high toughness, high deformation resistance, good corrosion performance and biological properties.

Stent implantation is currently the most effective and safe means to treat vascular stenosis. After more than 30 years of development, stent implantation and stent manufacturing technologies have basically matured. For products such as vascular stents, in order to achieve better clinical effects, a drug coating needs to be prepared on the stent surface, in order to inhibit the excessive proliferation of vascular tissue or to achieve a rapid endothelialization at the initial stage after implantation. The drug coating on the stent surface is generally obtained by ultrasonic atomization spraying. Coronary stents generally use a method of preparing a drug coating on the surface of a metal stent, and through balloon expansion, the stent is fixed to the target diseased part of the blood vessel, which acts as a long-term physical support for the diseased blood vessel and participates in the blood transportation in the blood vessel. However, after stent implantation, some patients still experience stent segment restenosis, thrombosis and late stent failure. The reasons may be related to the following factors: (1) During stent implantation and at the initial stage after implantation, the drug coating on the stent surface falls off and causes thrombus; (2) The long-term fatigue of the stent leads to the fracture or collapse of the stent (the current designed service life of a stent is 10 years); (3) The incidence of in-stent restenosis is higher in patients with metal allergy, and nickel allergy is most common in patients with metal allergy.

In order to further improve the safety and service life of the stent after implantation, and to avoid the stent segment restenosis caused by nickel allergy, material researchers have been working to develop metal materials for stents with higher biosafety and better mechanical properties. The Institute of Metal Research, Chinese Academy of Sciences has independently developed a high-nitrogen nickel-free stainless steel where the harmful nickel element is not added (Patent Document 1). Its composition is: Cr: 17-22%, Mn: 12-20%, Mo: 1-3%, Cu: 0.5-1.5%, N: 0.4-0.7%, Ni: ≤ 0.02%, C: ≤ 0.03%, Si: ≤ 0.75%, S: ≤ 0.01%, P: ≤ 0.025%, Fe: balance. The material has the characteristics such as high strength, high fatigue strength, high corrosion resistance and structure stability, and has obvious advantages as an implant material.

In terms of stent structure design and manufacturing technology, people are constantly pursuing reasonable matching of mechanical properties such as support strength of stents, so that stents have better clinical operability and clinical safety. In terms of improving the binding strength between a drug coating and a substrate, stent manufacturers use methods such as surface modification and preparation of gradient coatings to make the drug coating on the stent surface firmer. Northeastern University's "Metallic material surface texturing treatment method" (Patent Document 2), Sichuan University's "Titanium or titanium-alloy material with micron-nano coarse-structure surface and preparation method thereof" (Patent Document 3) and other techniques have certain effects for increasing the surface area and the binding firmness between the stent and the coating.

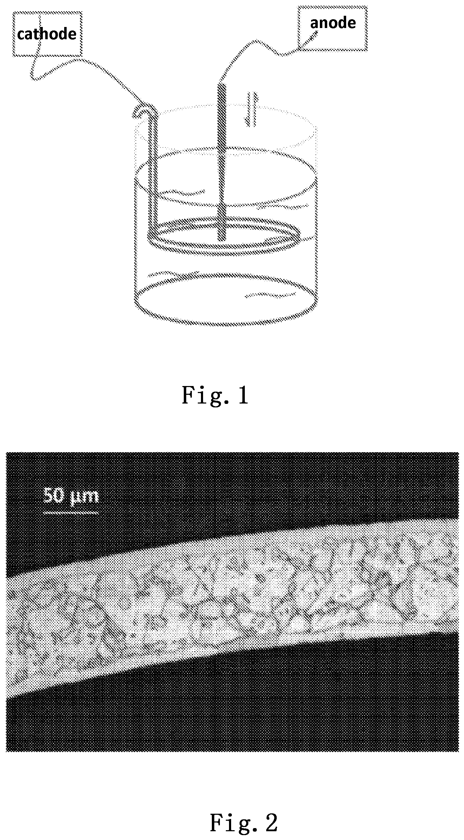

Electrochemical polishing is a relatively mature and commonly used metal polishing technology, especially suitable for polishing small samples, special-shaped samples and samples which cannot bear force. At present, small-caliber tubular mesh samples, such as vascular stents and other lumen stents for medical devices, and small springs for instrumentations, are all electrochemical-polished to improve surface quality and control size accuracy. Generally, this kind of sample is a hollowed-out structure, and the sample is only partially covered by a metal mesh wire structure, which is called metal coverage. The metal coverage of the small-caliber tubular mesh sample in the present invention is lower than 50%, so as to ensure sufficient solution exchange in the tube during the polishing. The method shown in Figure 1 is generally used for the polishing of small-caliber tubular mesh samples. Specifically, the sample is partially clamped with an anode, and the polishing of the sample is realized by reciprocating motion.

### Prior Art Documents

### Patent Documents

Patent Document 1: CN1519387A
Patent Document 2: CN101255593A
Patent Document 3: CN103668390A

### SUMMARY OF THE INVENTION

### TECHNICAL PROBLEM

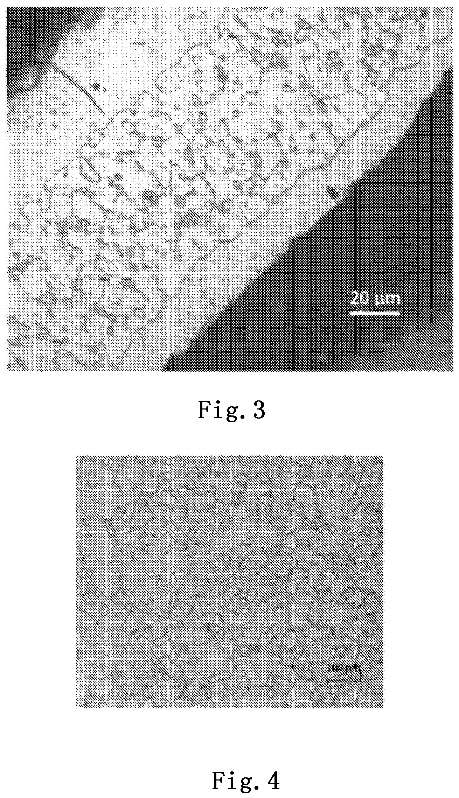

For chromium-manganese-nitrogen based high-nitrogen nickel-free austenitic stainless steel, due to the high saturated vapor pressure of manganese, when the material is subjected to high temperature heat treatment, manganese will volatilize from the free surface with lower binding force, forming a manganese-depleted layer on the surface. Figure 2 shows a metallographic photograph of a manganese-depleted layer formed on the surface of the tube. During the preparation of thin-walled tubes for vascular stents, with the increase of the number of heat treatments, the manganese-depleted layer on the surface of the tube continues to thicken. When the wall thickness and the manganese-depleted layer reach a certain ratio, the tube cracks. Figure 3 shows a metallographic photograph of cracks on the surface of the tube with severe manganese depletion.

In addition, the preparation of thin-walled tubes necessarily involves processes of both deformation and heat treatment. Due to the high deformation resistance of the material, it is difficult to achieve high size accuracy for tubes prepared by conventional processes, and cracks are prone to occur. Moreover, the ordinary heat treatment process will lead to the formation of a manganese-depleted layer on the surface of the tube, change the composition of the surface material, and result in a surface where a stable austenite cannot be formed, which will lead to cracking during deformation. Therefore, it is difficult for the product to meet the high precision and high stability requirements of the stainless steel tube in the field of vascular stents. In view of the above aspects, the high-nitrogen nickel-free stainless steel of Patent Document 1 still has room for improvement.

The surface treatment processes described in the above-mentioned Patent Documents 2 and 3 are difficult to obtain the desired effect for a tubular mesh stent with a mesh wire of only 0.08 to 0.1 mm. Therefore, there is an urgent need to find surface roughening techniques that are more suitable for stent manufacturing techniques.

In addition, for the existing polishing method of the small-caliber tubular mesh sample shown in the above-mentioned Figure 1, there are mainly the following problems: (1) Due to the contact problem between the sample and the electrode, a local current may be too large and the mesh wire may be broken; (2) Point contact leads to uneven polishing, especially when the sample is long, there will be a significant difference between the proximal electrode end and the distal electrode end. For springs and vascular stents, the above problems will directly lead to inconsistent mechanical properties of the product, and may even cause product failure.

In view of the above problems existing in the prior art, the purpose of the present invention is to provide a high-manganese (Mn ≥ 10% by weight), high-nitrogen (N: 0.7-1.3% by weight), nickel-free (Ni ≤ 0.05% by weight), austenitic stainless steel seamless thin-walled tube, and to provide a preparation method of the high-nitrogen nickel-free austenitic stainless steel seamless thin-walled tube with high size accuracy and controllable nitrogen and manganese contents. In addition, the purpose of the present invention is to provide a vascular stent with longer service life and higher safety and a manufacturing method thereof.

### TECHNICAL SOLUTION

In order to solve the above-mentioned problems, the inventors conducted in-depth research on the high-nitrogen nickel-free austenitic stainless steel seamless thin-walled tube used for the stent tube and the surface treatment method of the stent metal platform, and obtained the following (1)-(5) discoveries for the first time.
(1) According to high strength and high deformation resistance characteristics of the material, the use of single pass, multiple times of cold deformation with gradient decreasing can control the size accuracy of the tube and avoid the formation of micro-cracks.
(2) During the heat treatment, the surface layer of the tube can be free from manganese volatilization by applying a positive pressure of a protective atmosphere in the furnace, and meanwhile, the nitrogen content in the material and the overall performance of the tube can be regulated by applying a nitrogen partial pressure.
(3) After the heat treatment of the tube, mechanically remove the nitrogen-rich hard layer on the inner and outer surfaces caused by the heat treatment, and then perform the cold deformation of the next pass, which can prevent the tube from cracking and the introduction of foreign substances during the cold deformation process.
(4) The conduction between the electrode and the small-caliber tubular mesh sample (for example, the stent metal platform) is realized by rolling line contact, and the contact force and contact time between all points on the small-caliber tubular mesh sample and the electrode are uniform and consistent, so as to ensure the uniform polishing of the small-caliber tubular mesh sample, thereby realizing the surface finishing of the small-caliber tubular mesh sample and accurately controlling the mesh wire size.
(5) Furthermore, by utilizing the micro-potential difference between the electrode material and the small-caliber tubular mesh sample, the surface micro-patterning of the small-caliber tubular mesh sample can be simultaneously realized during the polishing process. In this way, under the premise of not introducing foreign substances and not affecting the overall performance of the sample, the surface micro-patterning improves the binding firmness of the small-caliber tubular mesh sample and the drug coating.

Therefore, using the high fatigue strength, high corrosion resistance, high structure stability and the characteristic of not containing a harmful nickel element for high-nitrogen nickel-free stainless steel materials, and further through the stent structure design and the polishing and roughening processes of the stent metal platform surface, vascular stents with longer service life and higher safety can be obtained.

The present invention has been completed based on the above findings, that is, the subject matters of the present invention are as follows.
1. A high-nitrogen nickel-free austenitic stainless steel seamless thin-walled tube, characterized in that,
   the N content is 0.7-1.3% by weight, and the tube is a single austenite structure in the solid solution state and in a cold deformation state of 66% or less, with a grain size of ≥ grade 7,
   said tube has a wall thickness of 60-200 µm, an outer diameter size deviation of ±0.03 mm, a wall thickness size deviation of ±0.02 mm, a yield strength of ≥ 600 MPa, a tensile strength of ≥ 1000 MPa, an axial elongation rate of ≥ 50%, and a pitting potential of ≥ 1000 mV.
2. The high-nitrogen nickel-free austenitic stainless steel seamless thin-walled tube according to above-mentioned 1, characterized in that, in % by weight, said tube has the following composition: Cr: 17-20%, Mn: 14-18%, Mo: 1-4%, N: 0.7-1.3%, Si: ≤ 0.75%, Cu: ≤ 0.25%, C: ≤ 0.03%, Si: ≤ 0.01%, P: ≤ 0.025%, Ni: ≤ 0.05%, and Fe: balance.
3. The high-nitrogen nickel-free austenitic stainless steel seamless thin-walled tube according to above-mentioned 1 or 2, characterized in that, said tube is used in the fields of medical devices, food and drug devices, jewelry, and instrumentations.
4. The high-nitrogen nickel-free austenitic stainless steel seamless thin-walled tube according to any one of above-mentioned 1 to 3, characterized in that, said tube is used for surgical implants.
5. The high-nitrogen nickel-free austenitic stainless steel seamless thin-walled tube according to above-mentioned 4, characterized in that, said surgical implants are human lumen stents.
6. The high-nitrogen nickel-free austenitic stainless steel seamless thin-walled tube according to above-mentioned 5, characterized in that, said human lumen stents are vascular stents.
7. A preparation method of the high-nitrogen nickel-free austenitic stainless steel seamless thin-walled tube according to any one of above-mentioned 1 to 6, characterized in that, through a combination of cold deformation and heat treatment of the high-nitrogen nickel-free austenitic stainless steel tube blank with a nitrogen content of < 0.7% by weight, no manganese is volatilized on the surface layer while molding the tube and controlling size accuracy, and the nitrogen content of the tube is increased,
   in a single pass, 2 to 3 times of cold deformation with a gradient decreasing are performed, the cumulative deformation amount of the pass is ≤ 50%, and the cold deformation amount of a single time is ≤ 30%,
   heat treatment is performed after the 2 to 3 times of cold deformation with the gradient decreasing in each pass, the temperature of said heat treatment is 1000-1150°C, and the treatment time is 5-90 minutes.
8. The preparation method according to above-mentioned 7, characterized in that, during said heat treatment, a positive pressure atmosphere of a mixed gas of argon and nitrogen is applied, the total pressure in a cold state is 0.12-0.30 MPa, and the nitrogen partial pressure is 5%-30%.
9. The preparation method according to above-mentioned 7 or 8, characterized in that, when the outer diameter of the tube is ≥ 3.0 mm, the cold deformation is performed 3 times in each pass, and the deformation amount of each time is sequentially 45-50%, 30-35% and 20-25% of the deformation amount of the pass; when the outer diameter of the tube is < 3.0 mm, the cold deformation is performed 2 times in each pass, and the deformation amount of each time is sequentially 55-60% and 40-45% of the deformation amount of the pass.
10. The preparation method according to any one of above-mentioned 7 to 9, characterized in that, the tube is subjected to the cold deformation of the next pass after mechanical removal of a nitrogen-rich hard layer on the inner and outer surfaces after the heat treatment.
11. A nickel-free metal drug-eluting vascular stent, characterized in that,
   the metal platform material of said stent is high-nitrogen nickel-free austenitic stainless steel, and in % by weight, the composition of the metal platform material is: Cr: 17-20%, Mn: 14-18%, Mo: 1-3 %, N: 0.8-1.2%, Si: ≤ 0.75%, Cu: ≤ 0.25%, C: ≤ 0.03%, Si: ≤ 0.01%, P: ≤ 0.025%, Ni: ≤ 0.05%, Fe: balance,
   said metal platform material has a tensile strength of 1100 MPa or more, a fatigue strength in the solid solution state of 570 MPa or more, and a fatigue strength at 20% cold deformation of 750 MPa or more,
   the pitting potential of said metal platform material in physiological saline and PBS buffer is 1000 mV or more, and
   when the cold deformation amount reaches 50%, said metal platform material still has a single austenite structure, with a grain size of ≥ grade 7.
12. The nickel-free metal drug-eluting vascular stent according to above-mentioned 11, wherein, the deformation amount of all deformation points of the said stent during crimping and expanding deformation is 15-25%, and the fatigue strength of the deformation part of the said stent is 750 MPa or more.
13. The nickel-free metal drug-eluting vascular stent according to above-mentioned 11 or 12, wherein, on the surface of the said stent metal platform, grains with different orientations form a micron-scale protrusion-recess structure, and the height difference between grains is 0.1-0.5 µm.
14. The nickel-free metal drug-eluting vascular stent according to any one of above-mentioned 11 to 13, which is used in heart or cerebral vessels.
15. The nickel-free metal drug-eluting vascular stent according to above-mentioned 14, which is used in coronary arteries.
16. A manufacturing method of the nickel-free metal drug-eluting vascular stent, characterized in that, during the preparation of the stent tube, through a combination of cold deformation and heat treatment of a high-nitrogen nickel-free austenitic stainless steel tube blank with a nitrogen content of < 0.7% by weight, the nitrogen content of the tube is increased to 0.8-1.2% and no manganese is volatilized on the surface layer while molding the tube and controlling size accuracy,
   in a single pass, 2 to 3 times of cold deformation with gradient decreasing are performed, the cumulative deformation amount of the pass is ≤ 50%, and the cold deformation amount of a single time is ≤ 30%,
   heat treatment is performed after the 2 to 3 times of cold deformation with the gradient decreasing in each pass, the temperature of said heat treatment is 1000-1150°C, and the treatment time is 5-90 minutes.
17. The manufacturing method of the nickel-free metal drug-eluting vascular stent according to above-mentioned 16, wherein, the temperature of said heat treatment is 1045-1055°C, the nitrogen partial pressure in the applied atmosphere is 5-30%, the balance is inert gas, and the pressure in the furnace is 1.5-3 atm.
18. The manufacturing method of the nickel-free metal drug-eluting vascular stent according to above-mentioned 16 or 17, wherein, when the outer diameter of the tube is ≥ 3.0 mm, the cold deformation is performed 3 times in each pass, and the deformation amount of each time is sequentially 45-50%, 30-35% and 20-25% of the deformation amount of the pass; when the outer diameter of the tube is < 3.0 mm, the cold deformation is performed 2 times in each pass, and the deformation amount of each time is sequentially 55-60% and 40-45% of the deformation amount of the pass.
19. The manufacturing method of the nickel-free metal drug-eluting vascular stent according to any one of above-mentioned 16 to 18, wherein, the tube is cut into a stent metal platform using a laser, and rolling line contact type electrochemical polishing is used so that said stent metal platform and a metal electrode are continuously in rolling line contact, the surface finishing of the stent metal platform is performed by controlling the rolling speed so as to control the thinning and breaking speed of the polishing solution film at the protrusions on the surface of the stent metal platform,
   meanwhile, said metal electrode is selected to be a dissimilar inert metal material to that of said stent metal platform, so that said metal electrode and said stent metal platform are conducted in a continuous rolling line contact mode, the surface of the stent metal platform forms a micron-scale protrusion-recess structure by means of grains with different orientations through the micro-potential difference between said metal electrode and said stent metal platform, and the height difference between grains is 0.1-0.5 µm.
20. The manufacturing method of the nickel-free metal drug-eluting vascular stent according to above-mentioned 19, wherein, during rolling line contact type electrochemical polishing, the current density is controlled at 0.8-1.0 A/cm².
21. The manufacturing method of the nickel-free metal drug-eluting vascular stent according to above-mentioned 19 or 20, wherein, during rolling line contact type electrochemical polishing, the electrochemical treatment temperature is controlled at 10-40°C.
22. The manufacturing method of the nickel-free metal drug-eluting vascular stent according to any one of above-mentioned 19 to 21, wherein, during rolling line contact type electrochemical polishing, the composition of the electrochemical polishing solution includes perchloric acid, glacial acetic acid and corrosion inhibitor, and the volume ratio of perchloric acid and glacial acetic acid, that is, perchloric acid/glacial acetic acid, is 1:4 to 1:20, and the volume ratio of the corrosion inhibitor in the polishing solution is 2-8%.
23. The manufacturing method of the nickel-free metal drug-eluting vascular stent according to any one of above-mentioned 19 to 22, wherein, during rolling line contact type electrochemical polishing, the polishing rolling speed is controlled at 2-2.5 cm/s.
24. The manufacturing method of the nickel-free metal drug-eluting vascular stent according to any one of above-mentioned 19 to 23, wherein, said dissimilar inert metal material is platinum or tantalum.
25. The manufacturing method of the nickel-free metal drug-eluting vascular stent according to any one of above-mentioned 19 to 24, wherein, said dissimilar inert metal material is platinum.

### ADVANTAGEOUS EFFECTS

According to the present invention, a high-manganese high-nitrogen nickel-free austenitic stainless steel thin-walled tube with high size accuracy, high surface quality and excellent comprehensive properties and a preparation method thereof can be provided.

In addition, according to the present invention, the stent metal platform material adopts a high-nitrogen nickel-free stainless steel material with high fatigue performance and high corrosion performance obtained by stage-by-stage nitriding, so that the stent metal platform has high mechanical properties and high fatigue strength, thereby the stent has a longer fatigue life.

Moreover, the stent metal platform material adopts high-nitrogen nickel-free austenitic stainless steel. The harmful nickel element with allergenic and carcinogenic effects is not actively added in the material, and the material has excellent corrosion resistance, thus reducing the risk of restenosis caused by metal ion dissolution or nickel allergy after the degradation of drug coating on the stent surface.

The fatigue strength of the stent is further improved through the structural design of controlling the deformation amount of the deformation points of the stent, so that the service life of the stent is longer.

By using the rolling line contact type electrochemical polishing, the surface finishing of small-caliber tubular mesh metal samples such as stent metal platform can be quickly realized, thereby greatly improving the surface polishing efficiency and surface polishing quality of the stent metal platform. Moreover, through the method of rolling line contact type electrochemical polishing of the present invention, the precise size control of the stent metal platform can be realized, and the method can greatly reduce the rejection rate of such precise metal samples.

In addition, the surface of the stent metal platform is roughened by rolling line contact type electrochemical polishing, and a micron-scale protrusion-recess structure is formed through grains with different orientations, which increases the binding force between the stent metal platform and the drug coating, enabling the drug coating on the stent surface to better resist damage that may be caused by deformation and fatigue. Therefore, the coating is not easy to fall off during deformation, delivery and service of the stent, which reduces the risk of thrombosis in the initial stage after stent implantation. Furthermore, since the surface roughening method of the present invention does not need to introduce foreign substances, it is safer than the chemical surface roughening method using corrosion. In addition, the surface roughening method of the present invention does not have the problem of reducing fatigue life caused by physical surface roughening methods such as texturing and sandblasting.

Since the high-safety nickel-free metal drug-eluting vascular stent of the present invention has the above-mentioned characteristics of long life and low risk, it is expected to improve the quality of life of implanted patients and benefit the society.

### BRIEF DESCRIPTION OF THE DRAWINGS

Figure 1 is a schematic diagram showing a conventional polishing method for a small-caliber tubular mesh sample.
Figure 2 is a metallographic photograph of a manganese-depleted layer formed on the surface of the tube.
Figure 3 is a metallographic photograph of cracking on the surface of the tube with severe manganese depletion.
Figure 4 is a metallographic structure photograph showing the axial cross section of a Φ3.0×0.11 mm tube of Example 1. It is a metallographic structure photograph with a magnification of 100 times taken with a Zeiss Observer Z1M metallographic microscope according to the GB/T 6397-2017 metal average grain size measurement method.
Figure 5 is a metallographic structure photograph showing the axial cross section of a Φ1.8×0.09 mm tube of Example 2. It is a metallographic structure photograph with a magnification of 100 times taken with a Zeiss Observer Z1M metallographic microscope according to the GB/T 6397-2017 metal average grain size measurement method.
Figure 6 is a metallographic structure photograph showing the axial cross section of a Φ4.5×0.19 mm tube of Example 3. It is a metallographic structure photograph with a magnification of 100 times taken with a Zeiss Observer Z1M metallographic microscope according to the GB/T 6397-2017 metal average grain size measurement method.
Figure 7 is a diagram showing the structure of a stent having a 2.5 mm nominal diameter of Example 4.
Figure 8 is a schematic diagram showing a rolling line contact type electrochemical polishing apparatus of the present invention. Figure 8A is a front view of the polishing apparatus. Figure 8B is a top view of the polishing apparatus.
Figure 9 is a diagram showing the macroscopic and microscopic morphology of the surface of the high-nitrogen nickel-free stainless steel vascular stent after surface modification by the method of the present invention in Example 4. Figure 9A and Figure 9B are macroscopic morphology of the surface of the high-nitrogen nickel-free stainless steel vascular stent, wherein Figure 9B is a partial enlarged view of Figure 9A. Figure 9C is microscopic morphology of the surface of the high-nitrogen nickel-free stainless steel vascular stent.
Figure 10 is a graph showing the morphology of a 316L stainless steel vascular stent after surface finishing, precise molding, and surface micro-patterning in Example 5. Figure 10A is morphology of the surface of the 316L stainless steel vascular stent under the metallographic microscope. Figure 10B is microscopic morphology of the surface of the 316L stainless steel vascular stent.
Figure 11 is a diagram showing the structure of a stent having a 2.5 mm nominal diameter of Example 6.
Figure 12 is a diagram showing the structure of a stent having a 3.0 mm nominal diameter of Example 7.
Figure 13 is a diagram showing a scanning electron microscope photograph of the surface of the roughened stent metal platform of Example 7.
Figure 14 is a diagram showing a laser confocal photograph of the surface of the roughened stent metal platform of Example 7.
Figure 15 is a diagram showing a scanning electron microscope photograph of the stent surface coating after fatigue in Example 7.
Figure 16 shows the X-ray diffraction spectrums of the Φ12×1.1 mm high-nitrogen nickel-free stainless steel solid solution tube (N: 0.92% by weight) obtained after the cold deformation and heat treatment of the seventh pass in Example 3, and the high-nitrogen nickel-free stainless steel tubes after 21%, 43%, and 66% cold deformation thereof.

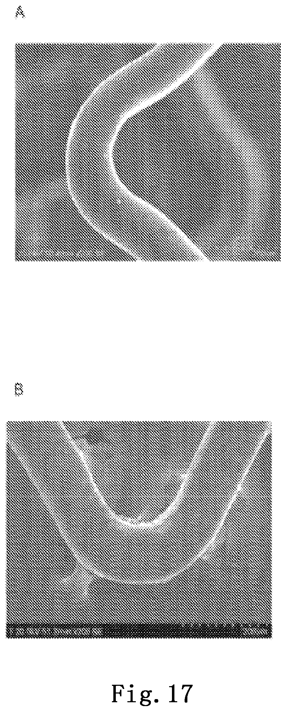

Figure 17 is a diagram showing the comparison results of the coating firmness of the stent metal platform surface after different surface treatments. Figure 17A shows the surface morphology of the drug coating on the stent metal platform surface after stent crimping and expanding, wherein the drug coating is sprayed on the surface after the roughening modification of the present invention. Figure 17B shows the surface morphology of the drug coating on the stent metal platform surface after stent crimping and expanding, wherein the drug coating is directly sprayed on the surface after conventional electrochemical polishing without roughening modification.

### DETAILED EMBODIMENTS

The present invention provides a high-nitrogen nickel-free austenitic stainless steel seamless thin-walled tube, characterized in that, the N content is 0.7-1.3% by weight, and the tube is a single austenite structure in the solid solution state and in a cold deformation state of 66% or less, with a grain size of grade 7 or more (including grade 7) (measured according to GB/T 6394-2002 metal average grain size measurement method), the wall thickness is 60-200 µm, the outer diameter size deviation is ±0.03 mm, the wall thickness size deviation is ±0.02 mm, the yield strength is ≥ 600 MPa, the tensile strength is ≥ 1000 MPa, the axial elongation rate is ≥ 50%, and the pitting potential is ≥ 1000 mV.

The above-mentioned high-nitrogen nickel-free austenitic stainless steel seamless thin-walled tube of the present invention preferably, in % by weight, has the following composition: Cr: 17-20%, Mn: 14-18%, Mo: 1-4%, N: 0.7-1.3%, Si: ≤ 0.75%, Cu: ≤ 0.25%, C: ≤ 0.03%, Si: ≤ 0.01%, P: ≤ 0.025%, Ni: ≤ 0.05%, and Fe: balance.

The above-mentioned high-nitrogen nickel-free austenitic stainless steel seamless thin-walled tube of the present invention is suitable for use in the fields such as medical devices, food and drug devices, jewelry, and instrumentations, preferably used for surgical implants. Said surgical implants are human lumen stents, more preferably vascular stents.

The present invention also provides a preparation method of the above-mentioned high-nitrogen nickel-free austenitic stainless steel seamless thin-walled tube, characterized in that, through a combination of cold deformation and heat treatment of the high-nitrogen nickel-free austenitic stainless steel tube blank with a nitrogen content of < 0.7% by weight, no manganese is volatilized on the surface layer while molding the tube and controlling size accuracy, and the nitrogen content of the tube is increased. In this preparation method, according to material properties, in a single pass, 2 to 3 times of cold deformation with gradient decreasing are performed, the cumulative deformation amount of the pass is ≤ 50%, and the cold deformation amount of a single time is ≤ 30%, thereby controlling size accuracy of the tube. Heat treatment is performed after the 2 to 3 times of cold deformation with the gradient decreasing in each pass, the temperature of said heat treatment is 1000-1150°C, and the treatment time is between 5-90 minutes depending on the charging amount of the furnace and the wall thickness of the tube.

In the above-mentioned preparation method of the present invention, preferably, during said heat treatment, a positive pressure atmosphere of a mixed gas of argon and nitrogen is applied, the total pressure in a cold state is 0.12-0.30 MPa, and the nitrogen partial pressure is 5%-30%. By adjusting the total pressure and nitrogen partial pressure of the protective atmosphere, the surface manganese can be prevented from volatilizing while the nitrogen content of the tube is controllable within the range of 0.7-1.3 wt %.

In the above-mentioned preparation method of the present invention, preferably, when the outer diameter of the tube is ≥ 3.0 mm, the cold deformation is performed 3 times in each pass, and the deformation amount of each time is sequentially 45-50%, 30-35% and 20-25% of the deformation amount of the pass; when the outer diameter of the tube is < 3.0 mm, the cold deformation is performed 2 times in each pass, and the deformation amount of each time is sequentially 55-60% and 40-45% of the deformation amount of the pass.

In the above-mentioned preparation method of the present invention, preferably, the tube is subjected to the cold deformation of the next pass after mechanical removal of a nitrogen-rich hard layer on the inner and outer surfaces after the heat treatment. Thereby, it is possible to prevent cracking of the tube and introduction of foreign substances during the next cold deformation.

The metal platform material of the high-safety nickel-free metal drug-eluting vascular stent of the present invention adopts high-nitrogen nickel-free austenitic stainless steel with high strength, high fatigue strength and high corrosion resistance, in % by weight, the composition thereof is: Cr: 17-20%, Mn: 14-18%, Mo: 1-3 %, N: 0.8-1.2%, Si: ≤ 0.75%, Cu: ≤ 0.25%, C: ≤ 0.03%, Si: ≤ 0.01%, P: ≤ 0.025%, Ni: ≤ 0.05%, Fe: balance.

In order to obtain the above-mentioned stent metal platform material of the present invention where the nitrogen content is high and the volatilization of manganese in the material has been suppressed, in the preparation process of the stent tube, while heat treatment eliminates cold deformation stress to achieve solid solution, stage-by-stage pressure nitriding increases the nitrogen content in the tube. Thus, the obtained stent metal platform material of the present invention can have a tensile strength of 1100 MPa or more and a fatigue strength of 570 MPa or more, much higher than the fatigue strength of the mainstream stent materials currently used clinically.

Through the design of the stent structure, the deformation amount of all the mesh wires of the stent during crimping and expanding deformation is 15-25%, so that the fatigue strength of the deformation part of the stent (long-term fatigue) is increased to 750 MPa or more. As a result, the risk of late fracture and collapse of the stent is reduced, the long-term safety and effectiveness of the stent in vivo is maximized, thereby the stent has a longer fatigue life, and the safe service period of the stent is prolonged.

In addition, the corrosion potential of the stent metal platform material of the present invention in physiological saline and PBS buffer solution can reach 1000 mV or more, and no passivation treatment is required to improve its surface corrosion resistance. Because the stent metal platform material of the present invention has excellent corrosion resistance and no harmful nickel element with allergenic and carcinogenic effects are added to the material, after the drug coating on the stent surface is degraded, the metal material has high biological safety, and the risk of late restenosis of the stent segment is reduced.

The tube is shaped by a laser cutting, and the surface finishing and size control of the stent metal platform are realized by means of rolling line contact type electrochemical polishing. In the rolling line contact type electrochemical polishing suitable for small-caliber tubular mesh metal samples of the present invention, stent metal platform and the metal electrode are continuously in rolling line contact. The surface finishing of the stent metal platform is performed quickly and uniformly by controlling the rolling speed so as to control the thinning and breaking speed of the polishing solution film at the protrusions on the surface of the stent metal platform. More preferably, in the rolling line contact type electrochemical polishing of the present invention, said metal electrode is selected to be a dissimilar inert metal material to that of said stent metal platform, so that said metal electrode and said stent metal platform to be polished are conducted in a continuous rolling line contact mode. Through the micro-potential difference between said metal electrode and said stent metal platform, the surface of the stent metal platform has a difference in polishing amount between grains, and thus the inner surface of the stent metal platform is micro-patterned.

The invention changes the surface treatment (surface polishing and surface roughening) technology of the existing small-caliber tubular mesh metal samples, from the traditional single-point and partial clamping to rolling line contact type electrochemical polishing. First, the contact type rolling polishing is used to accelerate the thinning and breaking speed of the polishing solution film at the protrusions on the surface of the sample, so as to accelerate the smoothing and polishing purpose of the protrusion parts on the surface, thus realizing the rapid surface finishing of the metal sample. Second, the rolling line contact type electrochemical polishing is used to avoid the unevenness of the metal sample caused by single-point and partial clamping polishing, and to avoid the phenomenon that the structure of the metal sample deviates from the target sample structure after polishing.

In addition, the metal electrode is selected to be dissimilar inert metal materials and conducts with the small-caliber tubular mesh metal sample to be polished in a continuous rolling line contact mode. Combined with an appropriate polishing voltage, through the micro-potential difference between the electrode and the metal piece to be polished, the surface of the small-caliber tubular mesh metal sample has a difference in polishing amount between grains, and thus the inner surface of the tubular mesh metal sample is micro-patterned, which increases the coating binding strength for the subsequent coating processing.

The rolling line contact type electrochemical polishing of the present invention is suitable for hollowed-out small-caliber tubular mesh metal samples including stents, the length of which is less than 80 mm, the tube diameter is less than 5 mm, and the metal coverage rate is less than 50%. In addition, the metal sample material includes: stainless steel, titanium alloy, cobalt-based alloy, magnesium alloy, iron alloy, zinc alloy, but not limited to the above alloys.

In the rolling line contact type electrochemical polishing of the present invention, the dissimilar inert metal material may be platinum or tantalum, preferably platinum.

In the rolling line contact type electrochemical polishing of the present invention, it is preferable to control the current density at 0.8-1.0 A/cm² and the polishing temperature at 10-40°C. By polishing at a low temperature of 10-40°C, the reaction speed can be reduced, and the controllability of the precise structure size is improved, which is beneficial to the uniform polishing. In addition, in the rolling line contact type electrochemical polishing of the present invention, it is preferable to use a polishing solution whose components include perchloric acid, glacial acetic acid, and a corrosion inhibitor. In the polishing solution, the volume ratio of perchloric acid and glacial acetic acid, that is, perchloric acid/glacial acetic acid, is preferably 1:4 to 1:20. In addition, the volume ratio of the corrosion inhibitor in the polishing solution is preferably 2-8%, more preferably 5%. The surface of the stent metal platform is made smooth by controlling the composition of the polishing solution, the polishing current density and the reaction temperature. Meanwhile, by controlling the electrode potential, the grains with different orientations have different polishing amounts, so as to realize the micro-roughening of the surface of the stent metal platform, forming a height difference of 0.1-0.5 µm, thereby increasing the binding force of the stent metal platform and the drug coating.

In addition, in the rolling line contact type electrochemical polishing of the present invention, it is preferable to control the rolling speed at 2-2.5 cm/s.

The rolling line contact type electrochemical polishing of the present invention can be applied not only to the surface modification of vascular stents, but also to the surface modification of peripheral stents, the surface modification of digestive tract stents, the surface modification of urinary system stents, the surface modification of large-sized metal conduits, and the surface modification of bone cages for bone filling, etc.

The drug coating that inhibits the proliferation of smooth muscle cells is prepared on the stent surface by ultrasonic atomization spraying. By controlling the spraying process and the surface roughening of the stent metal platform, the drug coating on the stent surface binds with the substrate in high strength. The drug is preferably rapamycin and its derivatives. Therefore, the coating will not be damaged or fall off during the mounting, delivery and expansion of the stent, and the coating will not be severely damaged due to fatigue of the stent and flushing of blood flow before the stent is wrapped in the endothelium, thereby reducing the risk of thrombosis in the initial stage after stent implantation.

The high-safety nickel-free metal drug-eluting vascular stent of the present invention can be used for heart and cerebral vessels and other arterial and venous vessels, and is preferably used for coronary arteries.

Hereinafter, the present invention will be described in detail based on examples. However, the examples are merely illustrative of the present invention, and do not limit the scope of the present invention.

### Example 1 High-nitrogen nickel-free austenitic stainless steel seamless thin-walled tube 1

A high-nitrogen nickel-free stainless steel as-forged bar with a nitrogen content of 0.62% by weight and a manganese content of 15.4% by weight was taken and processed by a deep hole drilling machine to obtain a tube blank with a size of Φ30×6 mm. The size of the designed finished tube was Φ3.0×0.11 mm. The number of cold deformation passes was 17, and the deformation amount of each pass was 40-50%. Each pass included three times of cold deformations, and the deformation amount of each time was sequentially 45-50%, 30-35% and 20-25% of the deformation amount of the pass. After cold deformations of each pass, ultrasonic cleaning was performed on the surface of the tube to remove the surface lubricant. After drying, it was put into a heat treatment furnace tank that can be evacuated and pressurized. The furnace tank material was 2520 high-temperature alloy, and there were three temperature measuring thermocouples inside to monitor the temperature in real time. After the furnace tank was evacuated to 10⁻¹ Pa, pumping was continuously performed for 10 minutes or more, and the valve of the vacuum system was closed. The furnace tank was filled with a mixed gas of nitrogen and argon, such that the total pressure was 0.15 MPa, and the ratio of nitrogen and argon was 1:9, that is, the nitrogen partial pressure was 10%. When the heating furnace temperature reached 1100°C, the furnace tank was sent into the tubular heating furnace, and timing was started when the furnace tank temperature reached 1100°C and became stable. The temperature holding time was determined depending on the charging amount of the furnace and the wall thickness of the tube, ranging between 5-60 minutes. After heat treatment of each pass, the inner and outer surfaces of the tube were mechanically ground and polished.

The inspection results of the finished tube are as follows: the outer diameter was 3.0±0.02 mm, the wall thickness was 0.11±0.01 mm, the nitrogen content was 0.81% by weight, the manganese content was 15.42% by weight, the yield strength was 608 MPa, the tensile strength was 1019 MPa, the axial elongation rate was 51%, and the pitting potential was 1000 mV. Among them, the measurement methods of the yield strength, the tensile strength and the elongation rate are as follows: according to GB/T 228.1-2010 Tensile Test of Metallic Materials Part 1: Test Method at Room Temperature, Z150 mechanical testing machine was used to conduct tensile test on metal tubes. The metallographic structure of the axial cross section of the tube is shown in Figure 4, which is a single austenite structure with a grain size of ≥ grade 7. And, according to the "GB/T3505-2009, GB/T1031-2009, GB/T10610-2009" standards, the Alpha-Step IQ contact surface morphology instrument was used to measure the inner and outer surface roughness of the tube, and the measurement results were Ra inner = 0.046 µm,Ra outer = 0.039 µm, respectively.

### Example 2 High-nitrogen nickel-free austenitic stainless steel seamless thin-walled tube 2

A high-nitrogen nickel-free stainless steel as-forged bar with a nitrogen content of 0.62% by weight and a manganese content of 15.4% by weight was taken and processed by a deep hole drilling machine to obtain a tube blank with a size of Φ30×6 mm. The size of the designed finished tube was Φ1.8×0.09 mm. The number of cold deformation passes was 21, and the deformation amount of each pass was 40-50%. When the outer diameter of the tube is ≥ 3.0 mm, the cold deformation was performed 3 times in each pass, and the deformation amount of each time was sequentially 45-50%, 30-35% and 20-25% of the deformation amount of the pass; when the outer diameter of the tube was <3.0 mm, the cold deformation was performed 2 times in each pass, and the deformation amount of each time was sequentially 55-60% and 40-45% of the deformation amount of the pass. After cold deformations of each pass, ultrasonic cleaning was performed on the surface of the tube to remove the surface lubricant. After drying, it was put into a heat treatment furnace tank that can be evacuated and pressurized. The furnace tank material was 2520 high-temperature alloy, and there were three temperature measuring thermocouples inside to monitor the temperature in real time. After the furnace tank was evacuated to 10⁻¹ Pa, pumping was continuously performed for 10 minutes or more, and the valve of the vacuum system was closed. The furnace tank was filled with a mixed gas of nitrogen and argon, such that the total pressure was 0.25 MPa, and the ratio of nitrogen and argon was 1:4, that is, the nitrogen partial pressure was 20%. When the heating furnace temperature reached 1050°C, the furnace tank was sent into the tubular heating furnace, and timing was started when the furnace tank temperature reached 1050°C and became stable. The temperature holding time was determined depending on the charging amount of the furnace and the wall thickness of the tube, ranging between 5-60 minutes. After heat treatment of each pass, the inner and outer surfaces of the tube were mechanically ground and polished.

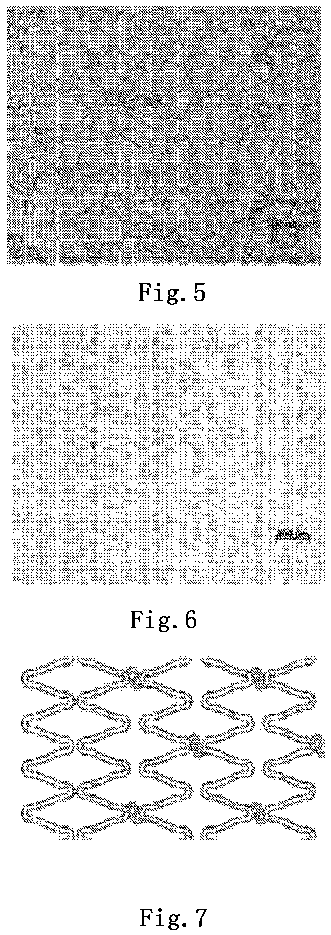

The inspection results of the finished tube are as follows: the outer diameter was 1.8±0.02 mm, the wall thickness was 0.09±0.01 mm, the nitrogen content was 1.15% by weight, the manganese content was 15.45% by weight, the yield strength was 781 MPa, the tensile strength was 1215 MPa, the axial elongation rate was 56%, and the pitting potential was 1090 mV. Among them, the measurement methods of the yield strength, the tensile strength and the elongation rate were the same as in Example 1. The metallographic structure of the axial cross section of the tube is shown in Figure 5, which is a single austenite structure with a grain size of ≥ grade 7. And, according to the roughness measurement method described in Example 1, the inner surface roughness of the tube Ra inner = 0.07 µm, and outer surface roughness of the tube Ra outer = 0.05 µm.

### Example 3 High-nitrogen nickel-free austenitic stainless steel seamless thin-walled tube 3

A high-nitrogen nickel-free stainless steel as-forged bar with a nitrogen content of 0.62% by weight and a manganese content of 15.4% by weight was taken and processed by a deep hole drilling machine to obtain a tube blank with a size of Φ30×6 mm. The size of the designed finished tube was Φ4.5×0.19 mm. The number of cold deformation passes was 15, and the deformation amount of each pass was 40-50%. Each pass included three times of cold deformations, and the deformation amount of each time was sequentially 45-50%, 30-35% and 20-25% of the deformation amount of the pass. After cold deformations of each pass, ultrasonic cleaning was performed on the surface of the tube to remove the surface lubricant. After drying, it was put into a heat treatment furnace tank that can be evacuated and pressurized. The furnace tank material was 2520 high-temperature alloy, and there were three temperature measuring thermocouples inside to monitor the temperature in real time. After the furnace tank was evacuated to 10⁻¹ Pa, pumping was continuously performed for 10 minutes or more, and the valve of the vacuum system was closed. The furnace tank was filled with a mixed gas of nitrogen and argon, such that the total pressure was 0.30 MPa, and the ratio of nitrogen and argon was 1:3, that is, the nitrogen partial pressure was 25%. When the heating furnace temperature reached 1100°C, the furnace tank was sent into the tubular heating furnace, and timing was started when the furnace tank temperature reached 1100°C and became stable. The temperature holding time was determined depending on the charging amount of the furnace and the wall thickness of the tube, ranging between 15-60 minutes. After heat treatment of each pass, the inner and outer surfaces of the tube were mechanically ground and polished.

The inspection results of the finished tube are as follows: the outer diameter was 4.5±0.02 mm, the wall thickness was 0.19±0.01 mm, the nitrogen content was 1.08% by weight, the manganese content was 15.41% by weight, the yield strength was 711 MPa, the tensile strength was 1112 MPa, the axial elongation rate was 55%, and the pitting potential was 1040 mV. Among them, the measurement methods of the yield strength, the tensile strength and the elongation rate were the same as in Example 1. The metallographic structure of the axial cross section of the tube is shown in Figure 6, which is a single austenite structure with a grain size of ≥ grade 7. And, according to the roughness measurement method described in Example 1, the inner surface roughness of the tube Ra inner = 0.058 µm, and outer surface roughness of the tube Ra outer = 0.053 µm.

### Example 4 Surface finishing and precise molding of the high-nitrogen nickel-free stainless steel vascular stent

### (1) Surface pretreatment of the high-nitrogen nickel-free stainless steel vascular stent before polishing

The high-nitrogen nickel-free austenitic stainless steel seamless thin-walled tube of Example 2 was cut into coronary stents by laser. The stent structure is shown in Figure 7. The crimped diameter of the stent on the balloon was 0.9 mm, and the expanded diameter of the stent was 2.5 mm. The stent needs to be pretreated by pickling before polishing to remove the oxide layer produced by laser processing on the stent surface. The goal of pretreatment is to completely remove the oxide layer on the stent surface, so as to avoid the barrier of the oxide layer to the exchange of polishing solution during the electrochemical polishing. The pickling solution was a solution mainly composed of sulfuric acid and hydrogen peroxide. During the pickling process, the temperature of the pickling solution was controlled at 10-50°C. The stent was rinsed with plenty of running water after pickling to remove the residual pickling solution on the stent surface.

### (2) Surface finishing and precise molding of the high-nitrogen nickel-free stainless steel vascular stent

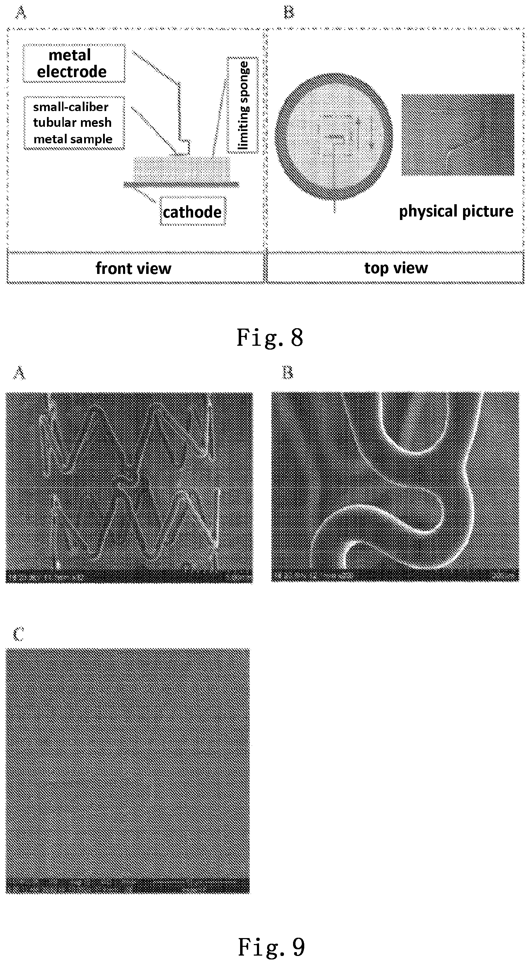

The surface finishing and precise molding of the stent are realized by the electrochemical polishing of the present invention. The schematic diagram of the electrochemical polishing apparatus is shown in Figure 8. As shown in the figure, the electrochemical polishing apparatus of the present invention is represented by Figure 8A (a front view of the polishing apparatus) and Figure 8B (a top view of the polishing apparatus). The apparatus is mainly composed of five parts, (1) the polishing tank, which is made of polypropylene or glass, and the size can be adjusted according to the size of the piece to be polished; (2) the cathode plate, which is made of a stainless steel disc, located at the bottom of the polishing tank; (3) Limiting sponge, made of polypropylene sponge or melamine sponge, etc., used to limit the distance between the piece to be polished and the cathode; (4) polishing hanger or polishing fixture, made of platinum filament, with a size of 0.8-1.2 mm depending on the inner diameter of the tube to be polished; (5) polishing solution, which immerses the cathode plate, the limiting sponge and the polishing hanger, and the piece to be polished remains completely immersed in the polishing solution during the rolling polishing of the piece to be polished.

In the electrochemical polishing of the present invention, the polishing solution was a mixture of perchloric acid, glacial acetic acid and a corrosion inhibitor. Among them, the volume ratio of perchloric acid to glacial acetic acid was 1:4, the corrosion inhibitor accounted for 2%-8% of the total volume of the polishing solution, the polishing temperature was 15°C, the cathode plate material was stainless steel, the metal electrode material was platinum, and the polishing voltage was 15 V depending on the size of the stent.

The specific polishing operation is as follows: the cathode was placed at the bottom of the container containing the electrochemical polishing solution, a porous sponge-like limiting plate was placed on the cathode, a platinum wire with a diameter of 0.9 mm passed through the stent, and the stent rolled at a constant speed with a linear speed of 20 mm/s on the limiting plate by moving the platinum wire, and stopped after reaching the preset polishing effect. After cleaning the stent with pure water, the residual acidic polishing solution on the stent surface was neutralized with NaOH solution. The polished stent should have a smooth surface, a uniform stent mesh wire structure, and meet the nominal weight requirements of the stent, so as to achieve the surface finishing and precise molding of the stent.

### (3) Morphology of high-nitrogen nickel-free stainless steel vascular stent after surface finishing and precise molding

Figure 9 shows the surface morphology of the high-nitrogen nickel-free stainless steel vascular stent after surface finishing and precise molding of this Example. It can be seen from the Figure that the surface modification method of the present invention can make the surface of the high-nitrogen nickel-free stainless steel vascular stent uniformly polished, thus it is suitable for surface finishing and precise molding of the vascular stent.

### Example 5 Surface finishing, precise molding and surface micro-patterning of the 316L stainless steel vascular stent

### (1) Surface pretreatment of the 316L stainless steel vascular stent before polishing

The 316L stainless steel vascular stent needs to be pretreated by pickling before polishing to remove the oxide layer produced by laser processing on the stent surface. The goal of pretreatment is to completely remove the oxide layer on the stent surface, so as to avoid the barrier of the oxide layer to the exchange of polishing solution during the electrochemical polishing. The pickling solution was a solution mainly composed of nitric acid and hydrofluoric acid. During the pickling process, the temperature of the pickling solution was controlled at 10-50°C. The stent was rinsed with plenty of running water after pickling to remove the residual pickling solution on the stent surface.

### (2) Surface finishing, precise molding and surface micro-patterning of the 316L stainless steel vascular stent

The surface finishing and precise molding of the stent were realized by electrochemical polishing, and the specific electrochemical polishing method was the same as Example 4. The polishing conditions such as the composition of the polishing solution, the rolling speed, and the polishing time were the same as those in Example 4. The polishing temperature was 15°C, the cathode plate material was stainless steel, the metal electrode material was platinum, and the polishing voltage was 25 V, depending on the size of the stent. The polishing process adopted the mode of continuous line contact rolling polishing. The polished stent should meet the nominal weight requirements of the stent, and a grain oriented micro-pattern should appear on the stent surface, so as to realize the surface finishing, precise molding and surface micro-patterning of the stent.

### (3) Morphology of the 316L stainless steel vascular stent after surface finishing and precise molding

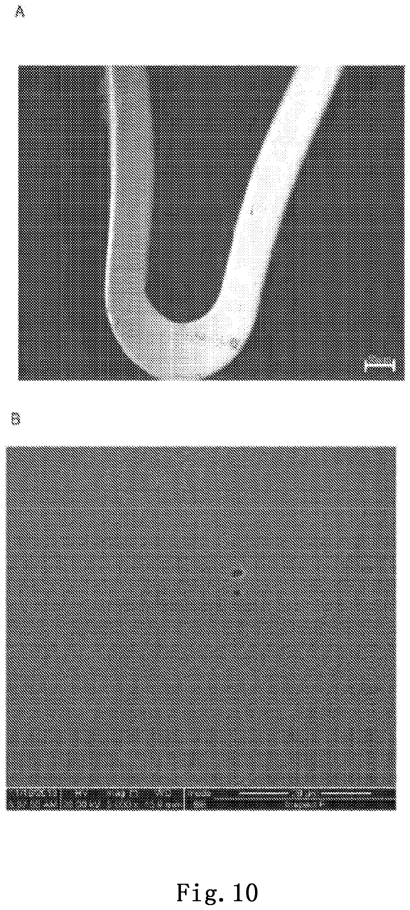

Figure 10 shows the surface morphology of the 316L stainless steel vascular stent after surface finishing and precise molding of this Example. It can be seen from the figure that the surface modification method of the present invention can make the surface of the 316L stainless steel vascular stent uniformly polished and meanwhile realize micro-patterning, thus it is suitable for surface finishing, precise molding and micro-patterning of the vascular stent.

### Example 6

Using the high-nitrogen steel bar with the composition shown in Table 1, the stent tube was prepared by the nitriding method described in Example 2, and the tube blank size was Φ30×6 mm. The number of cold deformation passes was 21. The furnace pressure was 0.25 MPa, the nitrogen partial pressure was 20%, the heat treatment temperature was 1050°C, and the temperature holding time was 30-5 minutes during heat treatment. After heat treatment of each pass, the inner and outer surfaces of the tube were mechanically ground and polished. According to GB/T 20124 Steel - Determination of nitrogen content - inert gas fusion thermal conductivity method (conventional method), the nitrogen content in the tube was measured with a TCH600 nitrogen, hydrogen and oxygen analyzer, and the nitrogen content of the finished tube was measured as 1.10% by weight. According to GB/T 228.1-2010 Tensile Test of Metallic Materials Part 1: Test Method at Room Temperature, Z150 mechanical testing machine was used to conduct tensile test on the finished tube, and the yield strength of the finished tube was measured as 761 MPa, the tensile strength was 1215 MPa and the axial elongation rate was 56%. According to YY/T 1074 the pitting potential of stainless steel surgical implants, electrochemical corrosion analysis was performed with GAMRY Reference600 electrochemical work station and the pitting potential of tube was measured as 1090 mV.

**Table 1. Chemical composition of bar used in stent tube preparation**

| Elements | Cr | Mn | Mo | N | C | Si | S | P | Cu | Ni | Fe |
|---|---|---|---|---|---|---|---|---|---|---|---|
| Content (%, by weight) | 18.48 | 15.30 | 2.58 | 0.62 | 0.029 | 0.31 | 0.006 | 0.008 | < 0.01 | 0.022 | balance |

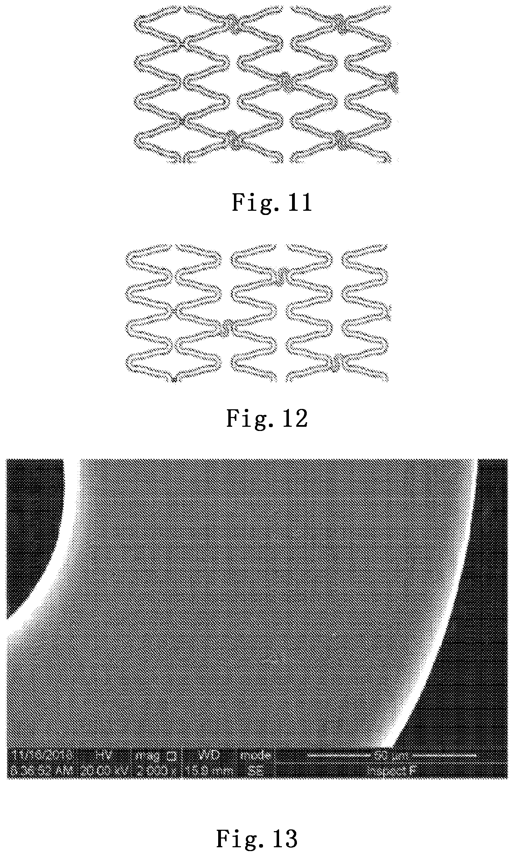

The tube was cut into a coronary stent with a laser, and its structure was shown in Figure 11. The crimped diameter of the stent on the balloon was 0.9 mm, and the expanded diameter of the stent was 2.5 mm. The mesh wire deformation amount of the stent during crimping and expanding was 15-25%. Through finite element analysis, the fatigue safety factor of stent was calculated as 3.77. According to "YY/T 0808-2010 Standard test methods for in vitro pulsatile durability of vascular stents", the fatigue performance of stent was tested with RDTL-0200 stent fatigue test system. The stent was released in a semi-compliant silicone tube matching the size of the stent. The working medium in the tube was PBS buffer at 37±2°C, and a pressure was applied inside the compliant tube in a pulsatile way. The minimum pressure was 75-80 mmHg, and the maximum pressure was 160-165 mmHg, the pulsation frequency was 45 Hz. After 570 million fatigue cycles (15 years of service life), no stent fracture and collapse were found.

### Example 7

Using the high-nitrogen steel bar with the composition shown in Table 2, the stent tube was prepared by the nitriding method under the following conditions, and the tube blank size was Φ30×6 mm. The number of cold deformation passes was 21. The furnace pressure was 0.25 MPa, the nitrogen partial pressure was 20%, the heat treatment temperature was 1050°C, and the temperature holding time was 30-5 minutes during heat treatment. After heat treatment of each pass, the inner and outer surfaces of the tube were mechanically ground and polished. The nitrogen content of the finished tube was 1.12% by weight, and according to the same method in Example 6, the yield strength was measured as 782 MPa, the tensile strength was measured as 1190 MPa, the axial elongation rate was measured as 54%, and the pitting potential of tube was measured as 1060 mV.

**Table 2. Chemical composition of bar used in stent tube preparation**

| Elements | Cr | Mn | Mo | N | C | Si | S | P | Cu | Ni | Fe |
|---|---|---|---|---|---|---|---|---|---|---|---|
| Content (%, by weight) | 18.03 | 16.00 | 2.33 | 0.64 | 0.028 | 0.28 | 0.004 | 0.005 | < 0.018 | 0.020 | balance |

The tube was cut into a coronary stent metal platform with a laser, and its structure is shown in Figure 12.

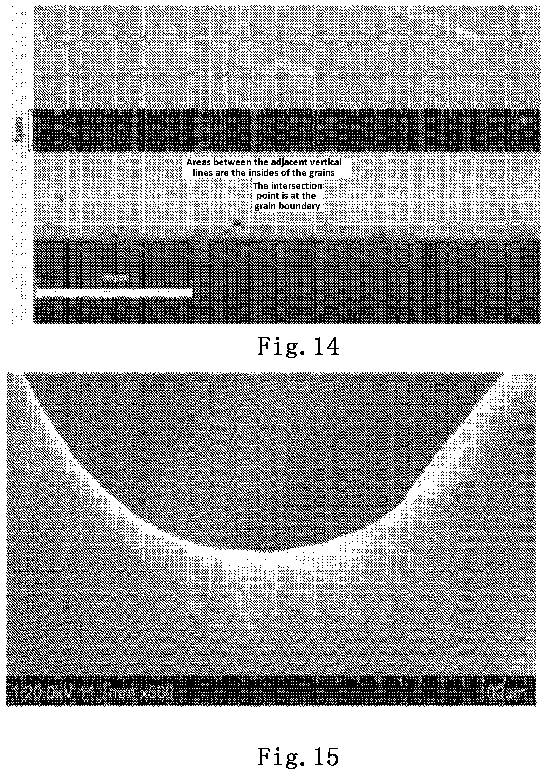

The obtained coronary stent metal platform was electrochemically modified as follows: the cathode was placed at the bottom of the container containing the electrochemical polishing solution, a porous sponge-like limiting plate was placed on the cathode, a platinum wire with a diameter of 0.9 mm passed through the stent, and the stent rolled at a constant speed with a linear speed of 20 mm/s on the limiting plate by moving the platinum wire, and stopped after reaching the preset polishing effect. After cleaning the stent with pure water, the residual acidic polishing solution on the stent surface was neutralized with NaOH solution to make the surface of the stent metal platform micro-roughened. The composition of the electrochemical polishing solution was perchloric acid and glacial acetic acid, the composition ratio was 1:10, the temperature of the electrochemical polishing solution was 35 ±2°C, and the electrochemical current density was 2.3 A/cm². Figure 13 and Figure 14 show the scanning electron microscope photograph and the laser confocal photograph of the surface of the stent metal platform after roughening treatment, respectively. According to the measurement, the surface height difference of the roughened stent metal platform was about 0.2 µm. Then, the rapamycin drug coating was sprayed on the surface of the stent by ultrasonic atomization. According to "YY/T 0808-2010 Standard test methods for in vitro pulsatile durability of vascular stents", the fatigue performance of the stent was tested with the RDTL-0200 stent fatigue test system. The stent was released in a semi-compliant silicone tube matching the size of the stent. The working medium in the tube was PBS buffer at 37±2°C, and a pressure was applied inside the compliant tube in a pulsatile way. The minimum pressure was 75-80 mmHg, and the maximum pressure was 160-165 mmHg, and the pulsation frequency was 1.2 Hz. Figure 15 shows the coating morphology of the coated stent after simulating pulsation and blood flow flushing in PBS buffer for 90 days. It can be seen from this result that the stent coating of the present invention does not fall off and is not damaged in a large area, and the coating has a high binding strength with the substrate.

### Experimental Example 1 Changes in mechanical properties before and after nitrogen increase

The mechanical properties of the high-nitrogen nickel-free stainless steel as-forged bar used in Example 1-3 and the finished tube of Example 1-3 obtained by high-temperature nitriding to further increase the nitrogen content of the material were measured, the measurement methods of yield strength, tensile strength, and elongation rate are as follows. According to GB/T 228.1-2010 Tensile Test of Metallic Materials Part 1: Test Method at Room Temperature, Z150 mechanical testing machine was used to conduct tensile test on metal tubes.

Table 3 summarizes the mechanical properties of the tube under different nitrogen contents. From the results, it can be seen that with the increase of nitrogen content, the strength of the material increases, and there is no substantial change in plasticity. That is, Examples 1-3 of the present invention obtain high manganese high-nitrogen nickel-free austenitic stainless steel thin-walled tubes with high size accuracy, high surface quality and excellent comprehensive properties.

**Table 3**

| | Nitrogen content (%, by weight) | Yield strength (MPa) | Tensile strength (MPa) | Elongation rate (%) |
|---|---|---|---|---|
| High-nitrogen nickel-free stainless steel as-forged bar | 0.62 | 480 | 900 | 54 |
| Finished tube of Example 1 | 0.81 | 608 | 1019 | 51 |
| Finished tube of Example 3 | 1.08 | 711 | 1112 | 55 |
| Finished tube of Example 2 | 1.15 | 781 | 1215 | 56 |

### Experimental Example 2 Structural changes before and after cold deformation

X-ray diffraction spectrums of the Φ12×1.1 mm high-nitrogen nickel-free stainless steel solid solution tube (N: 0.92% by weight) obtained after the cold deformation and heat treatment of the seventh pass in Example 3, and the high-nitrogen nickel-free stainless steel tubes after 21%, 43%, and 66% cold deformation thereof were measured. The specific measurement method is according to JY/T 009-1996 General rules for rotating target polycrystal X-ray diffractometry, Rigaku D/max 2500PC X-ray Diffractometer was used to measure the metal tube samples.

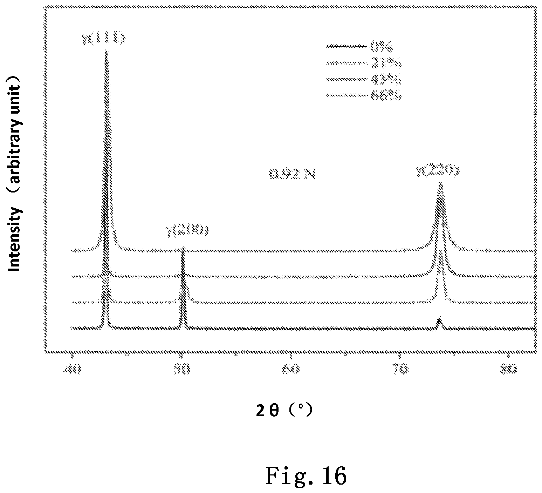

Figure 16 shows the X-ray diffraction spectrums of the high-nitrogen nickel-free stainless steel (N: 0.92% by weight) tubes in the solid solution state and in the above three cold deformation states, where X-ray diffraction spectrums of the (111) crystal plane, (200) crystal plane, and (220) crystal plane are standard austenite X-ray diffraction spectrums, and there is no shift in all diffraction peaks, indicating that the material remains a stable austenitic structure in the solid solution state and in a cold deformation state of less than 66%. That is, the austenite structure of the high-nitrogen nickel-free austenitic stainless steel thin-walled tube obtained by the present invention will not be affected in term of stability when used in a cold deformation state of less than 66%.

### Experimental Example 3 Comparison of coating firmness on the surface of stent metal platform after different surface treatments

The coating firmness index on the stent surface is an important evaluation index for drug coatings. The coating firmness evaluation method adopted in the present invention is as follows:
(1) Experimental grouping: Group I was the stent after conventional electrochemical polishing (that is, the high-nitrogen nickel-free stainless steel vascular stent after surface pretreatment before polishing in Example 4 after conventional electrochemical polishing), which was directly sprayed to form a drug coating, conventional electrochemical polishing referring to: the sample was partially clamped with an anode, and the polishing of the sample was realized by reciprocating motion; Group II was the stent after the surface roughening by the rolling line contact type electrochemical polishing of the present invention (that is, the high-nitrogen nickel-free stainless steel vascular stent after surface finishing and precise molding obtained in Example 4), which was directly sprayed to form a drug coating. After drying the stent for 1 day, it was sterilized by ethylene oxide and aerated for 7 days.
(2) The drug stents in Groups I and II were crimped and mounted using the special mounting equipment for stent system-vascular stent crimper to form a stent system.
(3) A pressure pump was used to expand the stent system mounted above respectively, with a nominal pressure of 12 atm. After the stent was unloaded, a scanning electron microscope observation was performed to observe particularly the coating morphology at the part with the largest deformation of the stent mesh wire.

Figure 17 shows the surface morphology of the stent metal platforms subjected to different surface treatments after being sprayed to form drug coatings followed by stent crimping and expanding. As shown in Figure 17B, when the drug coating is directly sprayed after conventional electrochemical polishing, the stent will partially fall off after expansion. On the other hand, as shown in Figure 17A, after the surface modification of the present invention, the coating has very good binding properties, and even after relatively large reciprocating deformations, the coating still maintains good shape characteristics.

## Claims

1. A high-nitrogen nickel-free austenitic stainless steel seamless thin-walled tube, **characterized in that**,
N content is 0.7-1.3% by weight, and the tube is a single austenite structure in a solid solution state and in a cold deformation state of 66% or less, with a grain size of ≥ grade 7,
said tube has a wall thickness of 60-200 µm, an outer diameter size deviation of ±0.03 mm, a wall thickness size deviation of ±0.02 mm, a yield strength of ≥ 600 MPa, a tensile strength of ≥ 1000 MPa, an axial elongation rate of ≥ 50%, and a pitting potential of ≥ 1000 mV.

2. The high-nitrogen nickel-free austenitic stainless steel seamless thin-walled tube according to claim 1, **characterized in that**, in % by weight, said tube has the following composition: Cr: 17-20%, Mn: 14-18%, Mo: 1-4%, N: 0.7-1.3%, Si: ≤ 0.75%, Cu: ≤ 0.25%, C: ≤ 0.03%, Si: ≤ 0.01%, P: ≤ 0.025%, Ni: ≤ 0.05%, and Fe: balance.

3. The high-nitrogen nickel-free austenitic stainless steel seamless thin-walled tube according to claim 1 or 2, **characterized in that**, said tube is used in the fields of medical devices, food and drug devices, jewelries, and instrumentations.

4. The high-nitrogen nickel-free austenitic stainless steel seamless thin-walled tube according to any one of claims 1 to 3, **characterized in that**, said tube is used for surgical implants.

5. The high-nitrogen nickel-free austenitic stainless steel seamless thin-walled tube according to claim 4, **characterized in that**, said surgical implants are human lumen stents.

6. The high-nitrogen nickel-free austenitic stainless steel seamless thin-walled tube according to claim 5, **characterized in that**, said human lumen stents are vascular stents.

7. A preparation method of the high-nitrogen nickel-free austenitic stainless steel seamless thin-walled tube according to any one of claims 1 to 6, **characterized in that**, through a combination of cold deformation and heat treatment of the high-nitrogen nickel-free austenitic stainless steel tube blank with a nitrogen content of < 0.7% by weight, no manganese is volatilized on the surface layer while molding the tube and controlling size accuracy, and the nitrogen content of the tube is increased,
in a single pass, 2 to 3 times of cold deformation with a gradient decreasing are performed, the cumulative deformation amount of the pass is ≤ 50%, and the cold deformation amount of a single time is ≤ 30%,
heat treatment is performed after the 2 to 3 times of cold deformation with the gradient decreasing in each pass, the temperature of said heat treatment is 1000-1150°C, and the treatment time is 5-90 minutes.

8. The preparation method according to claim 7, **characterized in that**, during said heat treatment, a positive pressure atmosphere of a mixed gas of argon and nitrogen is applied, the total gas pressure in a cold state is 0.12-0.30 MPa, and the nitrogen partial pressure is 5%-30%.

9. The preparation method according to claim 7 or 8, **characterized in that**, when the outer diameter of the tube is ≥ 3.0 mm, the cold deformation is performed 3 times in each pass, and the deformation amount of each time is sequentially 45-50%, 30-35% and 20-25% of the deformation amount of the pass; when the outer diameter of the tube is < 3.0 mm, the cold deformation is performed 2 times in each pass, and the deformation amount of each time is sequentially 55-60% and 40-45% of the deformation amount of the pass.

10. The preparation method according to any one of claims 7 to 9, **characterized in that**, the tube is subjected to the cold deformation of next pass after mechanical removal of a nitrogen-rich hard layer on the inner and outer surfaces after the heat treatment.

11. A nickel-free metal drug-eluting vascular stent, **characterized in that**,
the metal platform material of said stent is high-nitrogen nickel-free austenitic stainless steel, and in % by weight, the composition of the metal platform material is: Cr: 17-20%, Mn: 14-18%, Mo: 1-3 %, N: 0.8-1.2%, Si: ≤ 0.75%, Cu: ≤ 0.25%, C: ≤ 0.03%, Si: ≤ 0.01%, P: ≤ 0.025%, Ni: ≤ 0.05%, Fe: balance,
said metal platform material has a tensile strength of 1100 MPa or more, a fatigue strength in the solid solution state of 570 MPa or more, and a fatigue strength at 20% cold deformation of 750 MPa or more,
the pitting potential of said metal platform material in physiological saline and PBS buffer is 1000 mV or more, and
when the cold deformation amount reaches 50%, said metal platform material still has a single austenite structure, with a grain size of ≥ grade 7.

12. The nickel-free metal drug-eluting vascular stent according to claim 11, wherein, the deformation amount of all deformation points of the said stent during crimping and expanding deformation is 15-25%, and the fatigue strength of the deformation part of the said stent is 750 MPa or more.

13. The nickel-free metal drug-eluting vascular stent according to claim 11 or 12, wherein, on the surface of the said stent metal platform, grains with different orientations form a micron-scale protrusion-recess structure, and the height difference between grains is 0.1-0.5 µm.

14. The nickel-free metal drug-eluting vascular stent according to any one of claims 11 to 13, which is used in heart or cerebral vessels.

15. The nickel-free metal drug-eluting vascular stent according to claim 14, which is used in coronary arteries.

16. A manufacturing method of the nickel-free metal drug-eluting vascular stent, **characterized in that**, during the preparation of the stent tube, through a combination of cold deformation and heat treatment of a high-nitrogen nickel-free austenitic stainless steel tube blank with a nitrogen content of < 0.7% by weight, the nitrogen content of the tube is increased to 0.8-1.2% and no manganese is volatilized on the surface layer while molding the tube and controlling size accuracy,
in a single pass, 2 to 3 times of cold deformation with gradient decreasing are performed, the cumulative deformation amount of the pass is ≤ 50%, and the cold deformation amount of a single time is ≤ 30%,
heat treatment is performed after the 2 to 3 times of cold deformation with the gradient decreasing in each pass, the temperature of said heat treatment is 1000-1150°C, and the treatment time is 5-90 minutes.

17. The manufacturing method of the nickel-free metal drug-eluting vascular stent according to claim 16, wherein, the temperature of said heat treatment is 1045-1055°C, the nitrogen partial pressure in the applied atmosphere is 5-30%, the balance is inert gas, and the pressure in the furnace is 1.5-3 atm.

18. The manufacturing method of the nickel-free metal drug-eluting vascular stent according to claim 16 or 17, wherein, when the outer diameter of the tube is ≥ 3.0 mm, the cold deformation is performed 3 times in each pass, and the deformation amount of each time is sequentially 45-50%, 30-35% and 20-25% of the deformation amount of the pass; when the outer diameter of the tube is < 3.0 mm, the cold deformation is performed 2 times in each pass, and the deformation amount of each time is sequentially 55-60% and 40-45% of the deformation amount of the pass.

19. The manufacturing method of the nickel-free metal drug-eluting vascular stent according to any one of claims 16 to 18, wherein, the tube is cut into a stent metal platform using a laser, and rolling line contact type electrochemical polishing is used so that said stent metal platform and a metal electrode are continuously in rolling line contact, the surface finishing of the stent metal platform is performed by controlling the rolling speed so as to control the thinning and breaking speed of the polishing solution film at the protrusions on the surface of the stent metal platform,
meanwhile, said metal electrode is selected from a dissimilar inert metal material to that of said stent metal platform, so that said metal electrode and said stent metal platform are conducted in a continuous rolling line contact mode, the surface of the stent metal platform forms a micron-scale protrusion-recess structure by means of grains with different orientations through the micro-potential difference between said metal electrode and said stent metal platform, and the height difference between grains is 0.1-0.5 µm.

20. The manufacturing method of the nickel-free metal drug-eluting vascular stent according to claim 19, wherein, during rolling line contact type electrochemical polishing, current density is controlled at 0.8-1.0 A/cm².

21. The manufacturing method of the nickel-free metal drug-eluting vascular stent according to claim 19 or 20, wherein, during rolling line contact type electrochemical polishing, the electrochemical treatment temperature is controlled at 10-40°C.

22. The manufacturing method of the nickel-free metal drug-eluting vascular stent according to any one of claims 19 to 21, wherein, during rolling line contact type electrochemical polishing, the composition of the electrochemical polishing solution includes perchloric acid, glacial acetic acid and corrosion inhibitor, and the volume ratio of perchloric acid and glacial acetic acid, that is, perchloric acid/glacial acetic acid, is 1:4 to 1:20, and the volume ratio of the corrosion inhibitor in the polishing solution is 2-8%.

23. The manufacturing method of the nickel-free metal drug-eluting vascular stent according to any one of claims 19 to 22, wherein, during rolling line contact type electrochemical polishing, the polishing rolling speed is controlled at 2-2.5 cm/s.

24. The manufacturing method of the nickel-free metal drug-eluting vascular stent according to any one of claims 19 to 23, wherein, said dissimilar inert metal material is platinum or tantalum.

25. The manufacturing method of the nickel-free metal drug-eluting vascular stent according to any one of claims 19 to 24, wherein, said dissimilar inert metal material is platinum.
